# FASCICULE DE BREVET EUROPEEN

(11) **EP 2 324 779 B1**
(45) Date de publication et mention de la délivrance du brevet: **03.05.2017**
(21) Numéro de dépôt: 10190565.1
(22) Date de dépôt: 09.11.2010
(51) Int. Cl.: A61B 17/16, A61B 17/32

(54) **Dispositif chirurgical**
Chirurgische Vorrichtung
Surgical device

(30) Priorité: 09.11.2009 CH 17172009
(43) Date de publication de la demande: 25.05.2011
(73) Titulaire: Py, Jean-Pierre, 2900 Porrentruy (CH)
(72) Inventeur: Py, Jean-Pierre, 2900 Porrentruy (CH)
(74) Mandataire: Gevers SA

(56) Documents cités:
- US-A- 6 037 724
- US-A1- 2003 093 103
- US-A1- 2008 114 388

## Description

### Domaine technique

La présente invention concerne un dispositif chirurgical tel que défini dans la revendication 1.

### Etat de la technique

Le brevet US 6 037 724 décrit un dispositif chirurgical conforme au préambule de la revendication 1. Les dispositifs chirurgicaux sous forme d'instrument à main comprenant un moteur rotatif et un outil tel qu'un tournevis, une fraiseuse ou une scie sont connus dans le domaine médical sous le nom de moteurs de chirurgie. Ils sont apparus dès les années 1940 mais ils n'ont connu un réel développement qu'à partir des années 1970. Les moteurs de chirurgie sont aujourd'hui incontournables dans la chirurgie moderne. Ils sont utilisés, notamment, dans la chirurgie percutanée. Cette technique chirurgicale mini-invasive consiste à réaliser les interventions par de micro incisions. Auparavant, elles étaient pratiquées par de grandes ouvertures. On retrouve également les moteurs de chirurgie dans la chirurgie orthopédique. Ils répondent, en effet, aux besoins spécifiques liés à la chirurgie des extrémités. Un développement tout particulier a été réalisé dans le cas du traitement de l'Hallux Valgus plus communément appelé "oignon" du pied. Les applications et les indications nécessitant ces moteurs sont donc nombreuses, allant de la chirurgie de reconstruction à la chirurgie traumatologique.

Plus particulièrement, les moteurs de chirurgie sont utilisés pour sectionner, percer, décortiquer et lisser l'os et d'autres tissus associés lors de diverses procédures chirurgicales. Ils servent également à la mise en place ou à la coupe de vis, de broches et d'autres dispositifs de fixation. Ces instruments sont bien adaptés à un usage chirurgical grâce à leur ergonomie, leur légèreté, le couple et la puissance dont ils disposent. Le couple important de ces moteurs est particulièrement adapté aux coupes lors de la pose de prothèses mais également pour l'alésage et le fraisage à basse vitesse.

Il existe trois sortes de moteurs d'orthopédie. Une première sorte correspond aux moteurs pneumatiques. Ils fonctionnent avec une alimentation pneumatique. Ils se branchent au manomètre du détendeur d'une bouteille d'azote ou d'air comprimé. Une seconde sorte est représentée par les moteurs électriques. Ils fonctionnent avec une alimentation secteur par l'intermédiaire d'une console qui permet en général de brancher plusieurs moteurs de chirurgie. Une troisième sorte, plus récente, correspond aux moteurs autonomes. Ils fonctionnent avec des accumulateurs c'est à dire des batteries. Ces accumulateurs ont cependant une autonomie limitée, de 15 à 45 minutes selon l'effort demandé, et le temps de charge est en moyenne deux fois plus important que leur temps d'autonomie.

Le démarrage, l'arrêt et la vitesse de rotation du moteur sont commandés par le chirurgien directement sur l'instrument par l'intermédiaire d'un bouton poussoir tel qu'une gâchette, ou par l'intermédiaire d'un interrupteur à pédale.

Les modèles récents de moteur de chirurgie permettent un contrôle précis du couple et de la vitesse maximale de rotation par le biais de réglages déportés dans une console. Une infirmière a la responsabilité d'introduire les paramètres de réglages sur instructions du chirurgien en fonction du type d'intervention à pratiquer comme, par exemple, un alésage, un fraisage à basse vitesse, une coupe ou un perçage à haute vitesse. Le chirurgien ne peut effectuer le réglage lui-même, car la console, qui n'est pas stérile, est placée en zone non stérile dans la salle d'opération. Le réglage est alors effectué par une infirmière, sous les ordres du chirurgien.

Il existe alors un risque, en cas de non validation du programme sélectionné ou en cas d'incompréhension entre le chirurgien et l'infirmière, qu'un mauvais réglage tel qu'une vitesse maximale ou un couple trop élevés engendre des situations particulièrement dommageables pour le patient comme des nécroses des tissus.

Un but de la présente invention est donc de pallier ce risque. D'une façon plus générale, la présente invention a pour but de proposer un dispositif chirurgical dont les risques d'erreur humaine dans les réglages du moteur chirurgical sont réduits.

Un autre but de l'invention est de proposer un dispositif chirurgical permettant de donner une plus grande autonomie au praticien.

### Divulgation de l'invention

A cet effet, et conformément à la présente invention, il est proposé un dispositif chirurgical tel que défini dans la revendication 1.

Avantageusement, le dispositif peut comprendre en outre des moyens sécurisés de sélection manuelle agencés pour ajuster l'ensemble de paramètres d'asservissement sélectionné automatiquement en fonction du porte-outil, de manière à ajuster plus précisément encore, ces paramètres, à l'outil monté sur le porte-outil.

### Brève description des dessins

D'autres caractéristiques de la présente invention apparaîtront plus clairement à la lecture de la description qui va suivre, faite en référence au dessin annexé, dans lequel:
- la figure 1 représente une vue en perspective d'une première variante d'un instrument à main utilisé dans l'invention,
- la figure 2 représente une vue longitudinale d'un porte-outil de tournevis,
- la figure 3 représente une vue longitudinale d'un porte-outil de passe-broche,
- la figure 4 représente une vue longitudinale d'un porte-outil de scie sagittale équipé, et
- la figure 5 représente une vue longitudinale d'une deuxième variante d'un instrument à main utilisé dans l'invention.

### Mode(s) de réalisation de l'invention

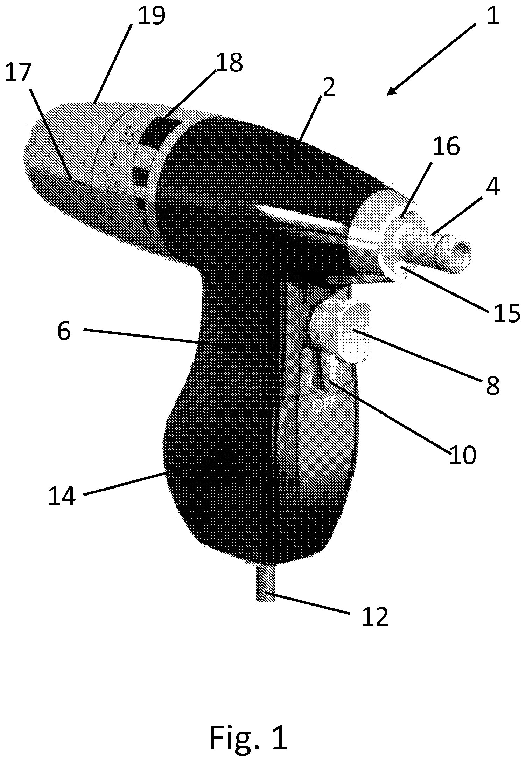

En référence à la figure 1, il est représenté un instrument à main 1 utilisé dans un dispositif selon l'invention. Ledit instrument à main 1 est muni d'un moteur rotatif 2 et d'un arbre d'entrainement 4 adapté pour accueillir une pièce à main amovible, non représentée. L'instrument comprend, en outre, une poignée 6 sensiblement perpendiculaire à l'axe d'entrainement 4. L'ergonomie générale de l'instrument est bien connue. Elle est choisie à dessein pour faciliter l'apprentissage des fonctionnalités supplémentaires, objets de l'invention. Par ailleurs, l'esthétique est soignée, les angles sont arrondis et la forme générale est homogène. Ces formes adaptées permettent une désinfection et un nettoyage aisés. Par ailleurs, l'instrument et ses outils peuvent être stérilisables à l'autoclave et lavables en machine.

L'instrument à main 1 est également muni d'un module électronique d'asservissement du moteur 2 comprenant un circuit électronique numérique.

L'asservissement s'applique avantageusement à un moteur électrique sans collecteur. Un exemple d'un asservissement d'un tel moteur est fourni, par exemple, dans le document WO 2005/037124A1.

Le module électronique permet donc, à tout moment, de contrôler la position du rotor, sa vitesse de rotation et son couple. Le moteur offre également une dynamique de fonctionnement très large, allant de vitesses très basses de quelques tours par minute (tr/min) jusqu'à plusieurs dizaines de milliers de tr/min, tout en garantissant, grâce à l'asservissement, une grande précision de la vitesse et du couple fournis sur l'arbre d'entrainement 4.

L'instrument 1 est donc agencé, dans ce mode de réalisation, pour que le chirurgien le tienne par une poignée 6 et agisse sur des moyens de commande de rotation du moteur. Ces moyens sont représentés ici par une commande de type gâchette 8 pour ajuster la vitesse de rotation du moteur et par un bouton rotatif 10 permettant de mettre l'instrument 1 hors-tension dans la position médiane du bouton rotatif 10, de choisir une rotation du moteur dans un sens horaire lorsque le bouton rotatif 10 est tourné à droite et dans un sens antihoraire lorsque le bouton rotatif 10 est tourné à gauche. D'autres types de boutons sont bien évidemment envisageables comme, par exemple, un bouton poussoir.

La source d'alimentation électrique est fournie via un raccord électrique flexible 12 relié à l'instrument 1. Un autre mode de réalisation selon l'invention comprend une source d'alimentation électrique fournie par une batterie amovible directement montée sur l'instrument 1. Elle occupe avantageusement la position marquée 14 dans la figure en s'intégrant à la poignée 6.

Un objet essentiel de l'invention est de réduire les risques de mauvais réglage de l'instrument et de rendre le chirurgien totalement maître de son intervention. Toutefois, les moyens de réglage doivent rester simples dans leurs ergonomies et leurs structures mécaniques de façon à faciliter l'adoption et l'usage de l'instrument, sa sécurité et sa stérilisation.

Par conséquent, l'instrument 1 comprend des moyens de détection automatique du type de porte-outil monté.

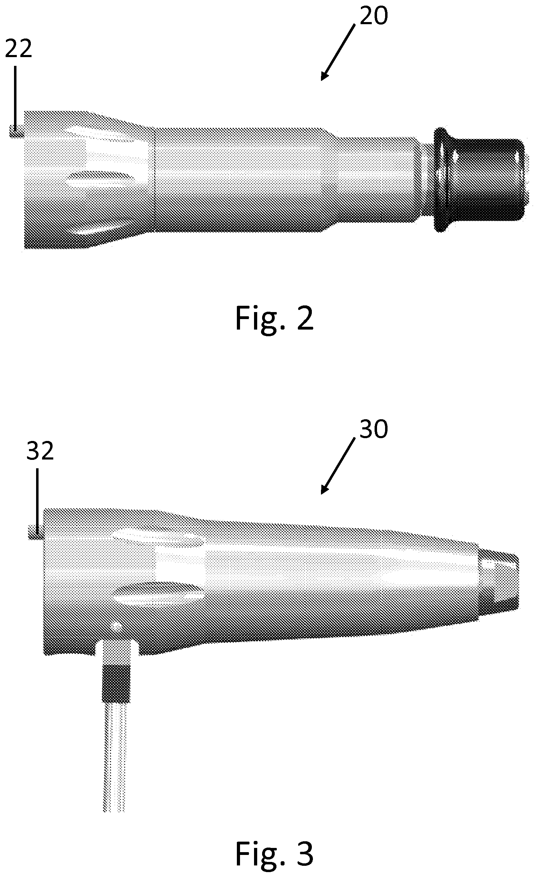

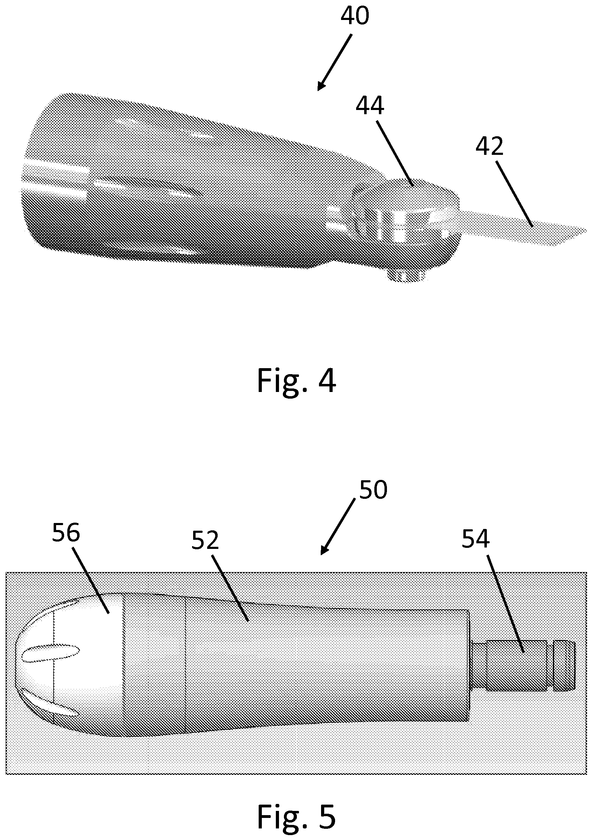

Il existe une variété de types de porte-outil adaptable sur l'arbre d'entrainement 4. Un premier type correspond aux opérations d'alésage et de fraisage. La vitesse de rotation du moteur est alors comprise entre 8000 tr/min et 16000 tr/min. Un second type de porte-outil 20, représenté sur la figure 2, correspond aux opérations de vissage et de dévissage, nécessitant, de plus, un arrêt automatique lorsqu'un couple maximal est atteint. La vitesse de rotation du moteur est alors comprise entre 5 tr/min et 100 tr/min. Un troisième type de porte-outil 30, représenté sur la figure 3, correspond au passe-broche. Enfin, un quatrième type de porte-outil 40, représenté sur la figure 4, permet les opérations de sciage par une scie sagittale 42, c'est-à-dire, une scie oscillante selon l'axe de rotation 44.

Chaque porte-outil induit un mode de fonctionnement particulier du moteur selon un gabarit déterminé. A ce mode de fonctionnement correspond un ensemble de paramètres d'asservissement du moteur, adapté à chaque porte-outil. Ainsi, un ensemble de paramètres d'asservissement comprend des vitesses de rotation minimale et maximale, des sens de rotation autorisés, du couple maximal autorisé dans un sens de rotation horaire, du couple maximal autorisé dans un sens de rotation anti-horaire et de l'action de maintien du couple ou d'arrêt du moteur lorsque le couple maximal est atteint.

Par ailleurs, le circuit électronique numérique du module d'asservissement est muni d'au moins une mémoire contenant les ensembles de paramètres d'asservissement du moteur, associés aux différents porte-outils utilisables.

Le circuit électronique numérique est agencé pour sélectionner automatiquement l'ensemble de paramètres d'asservissement en fonction du type de porte-outil détecté par les moyens de détection automatique.

Plus particulièrement, ces moyens de détection comportent au moins un ergot 22, 32, agencé sur le porte-outil 20, 30 (voir les Figures 2 et 3) pour coopérer mécaniquement avec au moins un évidement 16 du moteur (voir Figure 1) lorsque le porte-outil 20, 30 est monté sur l'arbre d'entrainement 4. Ces moyens de détection comprennent, en outre, au moins un élément aimanté agencé sur ledit porte-outil 20, 30 pour occuper une première position. Cette première position est associée à un type de porte-outil. Elle est référencée à la position de l'ergot 22, 32. Au moins un capteur 15 est agencé sur le moteur pour occuper une seconde position. Avantageusement, il s'agit d'un capteur à effet Hall ou magnétorésistif. Cette seconde position est référencée à la position de l'évidement 16 du moteur. Ainsi, lorsque le porte-outil 20, 30 est monté sur l'arbre d'entrainement 4, l'ergot 22, 32 s'engage dans l'évidement 16, fixant la position relative de l'élément aimanté et du capteur 15. Le circuit électronique est agencé pour traiter les signaux de sortie du capteur. Le traitement consiste à extraire des signaux la caractéristique dépendant de la position relative de l'élément aimanté par rapport au capteur. Le type de porte-outil monté est ainsi détecté et identifié.

La détection du porte-outil permet de restreindre avantageusement la dynamique du moteur à un certain mode de fonctionnement. Toutefois, le chirurgien doit pouvoir ajuster le couple et la vitesse précisément à l'outil et à l'opération. Par exemple, les vis employées ont des diamètres pouvant aller de 0,5 mm jusqu'à 3,5 mm et de 2 mm jusqu'à 5 mm pour les fraiseuses. L'ajustement précis du couple est donc nécessaire. Il en va de même pour certains implants sécables dont la pose nécessite ordinairement l'emploi de tournevis dynamométrique manuel.

Ainsi, la détection automatique du porte-outil est avantageusement complétée par des moyens sécurisés de sélection manuelle qui affinent le réglage. Ces moyens sont agencés pour ajuster l'ensemble de paramètres d'asservissement automatiquement sélectionné en fonction de l'outil et de l'opération.

La Figure 1 présente un mode de réalisation de ces moyens sécurisés de sélection manuelle. Ils comprennent un curseur 17, un ensemble de pictogrammes 18 représentant chacun une caractéristique d'un outil et une couronne montée en rotation sensiblement selon la direction de l'axe d'entrainement 4 du moteur 2 sur le côté opposé au montage de la pièce à main. Les moyens sont agencés pour que la couronne 19 en rotation aligne successivement le curseur 17 sur chaque pictogramme 18, de manière à sélectionner manuellement la caractéristique de l'outil et ajuster l'ensemble des paramètres d'asservissement sélectionné.

De plus, il est important de remarquer que ces moyens sont, en outre, agencés pour être compatibles avec une prise en main par un droitier ou par un gaucher. En effet, la Figure 1 présente l'instrument tel qu'il utilisé par un gaucher. Toutefois, un droitier en faisant tourner le curseur 17 jusque sur le côté non visible de l'instrument pourrait également utiliser l'instrument à sa main.

La Figure 5 présente un autre mode de réalisation de l'invention où l'instrument 50 présente la forme d'un crayon. Comme décrit ci-dessus, l'instrument 50 comprend un moteur rotatif 52 et un arbre d'entrainement 54 adapté pour accueillir une pièce à main amovible, telle que représentée sur les figures 2 à 4, ainsi qu'une couronne 56 correspondant à la couronne 19 du mode de réalisation précédent. Les pièces à main sont détectées par les mêmes moyens de détection que décrits ci-dessus. Les moyens de commande de rotation du moteur 52 sont avantageusement intégrés à une pédale reliée par un raccord électrique flexible à l'instrument.

Un autre mode de réalisation de l'invention, participant à un usage sécurisé du dispositif, consiste à ajouter des moyens de navigation qui sont utilisés pour la pose d'implant. Le système obtenu est spécialement adapté à la chirurgie du rachis, mais aussi de la hanche et du genou. Il permet de visser avec précision une vis dans un corps vertébral et d'en assurer sa fixation. Ces moyens de navigation comprennent un écran de visualisation de l'orientation 2D de l'implant, un émetteur de cette orientation 2D agencé sur l'instrument, un canal de transmission (l'information étant transmise, généralement, par un rayon infra rouge) et un récepteur. Par ailleurs, le couple de vissage est très précis. Il est déterminé par le fabricant de l'implant. L'invention offre donc avantageusement la possibilité d'agencer, sur l'instrument, l'émetteur des moyens de navigation et de contrôler précisément le couple de vissage. Le dispositif de l'invention peut également être utilisé en chirurgie dentaire, en particulier pour la pose d'implant.

Les différents modes de réalisation atteignent donc les objectifs de l'invention: offrir un dispositif chirurgical d'un emploi plus sûr.

## Revendications

1. Dispositif chirurgical comportant un instrument à main (1, 50) muni d'un moteur rotatif (2, 52), un arbre d'entrainement (4, 54) sur lequel est montée une pièce à main amovible comprenant un porte-outil (20, 30,40) et un outil, ledit instrument étant, de plus, muni
- d'un module électronique d'asservissement dudit moteur comprenant un circuit électronique numérique,
- de moyens de commande de rotation dudit moteur (2, 52),
- des moyens de détection automatique du type de porte-outil monté,
- et d'une source d'alimentation électrique (12),
ledit circuit électronique numérique étant muni d'au moins une mémoire contenant au moins deux ensembles de paramètres d'asservissement dudit moteur (2, 52), chaque ensemble de paramètres d'asservissement étant associé à un porte-outil (20, 30, 40), ledit circuit électronique numérique étant agencé pour sélectionner automatiquement l'ensemble de paramètres d'asservissement en fonction du type de porte-outil détecté par lesdits moyens de détection automatique, **caractérisé en ce que** les moyens de détection automatique du type de porte-outil comportent
- au moins un ergot (22, 32) agencé sur le porte-outil pour coopérer mécaniquement avec au moins un évidement (16) dudit moteur (2, 52) lorsque ledit porte-outil (20, 30, 40) est monté sur l'arbre d'entrainement (4, 54),
- au moins un élément aimanté agencé sur ledit porte-outil (20, 30, 40) pour occuper une première position associée à un type de porte-outil (20, 30, 40), ladite première position étant référencée à la position dudit ergot (22, 32),
- au moins un capteur (15) agencé sur ledit moteur (2, 52) pour occuper une seconde position référencée à la position dudit évidement,
et **en ce que** le circuit électronique est agencé pour traiter les signaux de sortie dudit capteur pour détecter le type de porte-outil monté en fonction de la position relative dudit élément aimanté avec ledit capteur.

2. Dispositif selon la revendication 1 **caractérisé en ce que** chaque ensemble de paramètres d'asservissement est associé à un porte-outil (20, 30, 40) et comprend des vitesses de rotation minimale et maximale, des sens de rotation autorisés, du couple maximal autorisé dans un sens de rotation horaire, du couple maximal autorisé dans un sens de rotation anti-horaire et de l'action de maintien du couple ou d'arrêt du moteur lorsque le couple maximal est atteint.

3. Dispositif selon l'une quelconque des revendications 1 ou 2, **caractérisé en ce que** le capteur est un capteur à effet Hall ou magnétorésistif.

4. Dispositif selon l'une quelconque des revendications 1 à 3, **caractérisé en ce qu'**il comprend en outre des moyens sécurisés de sélection manuelle agencés pour ajuster l'ensemble de paramètres d'asservissement automatiquement sélectionné.

5. Dispositif selon l'une quelconque des revendications 1 à 4, **caractérisé en ce que** ledit moteur (2, 52) est un moteur électrique sans collecteur.

6. Dispositif selon l'une quelconque des revendications 1 à 5, **caractérisé en ce que** ledit ensemble de paramètres d'asservissement du moteur comprend des vitesses de rotation minimale et maximale, des sens de rotation autorisés, un couple maximal autorisé dans un sens de rotation horaire, un couple maximal autorisé dans un sens de rotation anti-horaire et une action de maintien du couple ou d'arrêt du moteur lorsque le couple maximal est atteint.

7. Dispositif selon l'une quelconque des revendications 1 à 6, **caractérisé en ce que** les moyens de commande de rotation (8, 10) dudit moteur sont intégrés à l'instrument (1) et sont sélectionnés parmi le groupe comprenant un bouton poussoir, un bouton rotatif et une gâchette.

8. Dispositif selon l'une quelconque des revendications 1 à 6, **caractérisé en ce que** les moyens de commande de rotation dudit moteur (2, 52) sont intégrés à une pédale reliée par un raccord électrique flexible à l'instrument.

9. Dispositif selon l'une quelconque des revendications 4 à 8, **caractérisé en ce que** les moyens sécurisés de sélection manuelle comprennent un curseur (17), un ensemble de pictogrammes (18) représentant chacun une caractéristique d'un outil et une couronne (19, 56) montée en rotation sensiblement selon la direction de l'axe d'entrainement (4, 54) du moteur sur le côté opposé au montage de la pièce à main, lesdits moyens étant agencés pour que la couronne (19, 56) en rotation aligne successivement le curseur (17) sur chaque pictogramme (18), de manière à sélectionner manuellement la caractéristique de l'outil et ajuster l'ensemble des paramètres d'asservissement sélectionné, les moyens sécurisés de sélection étant en outre agencés pour être compatibles avec une prise en main par un droitier ou par un gaucher.

10. Dispositif selon l'une quelconque des revendications 1 à 9, **caractérisé en ce que** l'instrument (1) comprend en outre une poignée sensiblement perpendiculaire à l'axe d'entrainement.

11. Dispositif selon l'une quelconque des revendications 1 à 9, **caractérisé en ce que** l'instrument (50) a la forme d'un crayon.

12. Dispositif selon l'une quelconque des revendications 1 à 11 **caractérisé en ce qu'**il comprend des moyens de navigation IR agencés pour émettre une position 2D d'un implant.

13. Dispositif selon l'une quelconque des revendications 1 à 12, **caractérisé en ce que** la source d'alimentation électrique est fournie via un raccord électrique flexible (12) relié à l'instrument.

14. Dispositif selon l'une quelconque des revendications 1 à 12, **caractérisé en ce que** la source d'alimentation électrique est fournie par une batterie amovible montée sur l'instrument.

## Patentansprüche

1. Chirurgische Vorrichtung, aufweisend ein Handinstrument (1, 50), ausgestattet mit einem rotierenden Motor (2, 52), einer Antriebswelle (4, 54), auf welcher ein lösbares Handteil montiert ist, umfassend einen Werkzeughalter (20, 30, 40) und ein Werkzeug, wobei das Instrument ferner ausgestattet ist mit:
- einem elektronischen Steuermodul des Motors, umfassend einen digitalen elektronischen Kreis,
- Rotationssteuermitteln des Motors (2, 52),
- Mitteln zur automatischen Detektion des Typs des montierten Werkzeughalters,
- und einer elektrischen Versorgungsquelle (12),
wobei der digitale elektronische Kreis mit mindestens einem Speicher ausgestattet ist, der mindestens zwei Steuerparametergruppen des Motors (2, 52) enthält, wobei jede Steuerparametergruppe einem Werkzeughalter (20, 30, 40) zugeordnet ist, wobei der digitale elektronische Kreis ausgebildet ist, um die Steuerparametergruppe in Abhängigkeit vom Typ des von den Mitteln zur automatischen Detektion ermittelten Werkzeughalters automatisch auszuwählen,
**dadurch gekennzeichnet, dass** die Mittel zur automatischen Detektion des Werkzeughaltertyps aufweisen:
- mindestens einen Sporn (22, 32), der auf dem Werkzeughalter ausgebildet ist, um mit mindestens einer Aussparung (16) des Motors (2, 52) mechanisch zusammenzuwirken, wenn der Werkzeughalter (20, 30, 40) auf der Antriebswelle (4, 54) montiert ist,
- mindestens ein magnetisiertes Element, das auf dem Werkzeughalter (20, 30, 40) ausgebildet ist, um eine erste Position einzunehmen, die einem Werkzeughaltertyp (20, 30, 40) zugeordnet ist, wobei die erste Position zur Position des Sporns (22, 32) referenziert ist,
- mindestens einen Sensor (15), der auf dem Motor (2, 52) ausgebildet ist, um eine zweite Position einzunehmen, die zur Position der Aussparung referenziert ist,
und dass der elektronische Kreis ausgebildet ist, um die Ausgangssignale des Sensors zu verarbeiten, um den Werkzeughaltertyp zu ermitteln, der in Abhängigkeit von der relativen Position des magnetisierten Elements zum Sensor montiert ist.

2. Vorrichtung nach Anspruch 1, **dadurch gekennzeichnet, dass** jede Steuerparametergruppe einem Werkzeughaltertyp (20, 30, 40) zugeordnet ist und minimale und maximale Rotationsgeschwindigkeit, genehmigte Rotationsrichtungen, maximal zulässiges Moment in einer Rotationsrichtung im Uhrzeigersinn, maximal zulässiges Moment in einer Rotationsrichtung entgegen dem Uhrzeigersinn und Beibehaltungsaktion des Moments oder Stopp des Motors, wenn das maximale Moment erreicht ist, umfasst.

3. Vorrichtung nach einem der Ansprüche 1 oder 2, **dadurch gekennzeichnet, dass** der Sensor ein Sensor mit Hall-Effekt oder magnetresistent ist.

4. Vorrichtung nach einem der Ansprüche 1 bis 3, **dadurch gekennzeichnet, dass** sie ferner gesicherte manuelle Auswahlmittel umfasst, die ausgebildet sind, um die automatisch ausgewählte Steuerparametergruppe anzupassen.

5. Vorrichtung nach einem der Ansprüche 1 bis 4, **dadurch gekennzeichnet, dass** der Motor (2, 52) ein Elektromotor ohne Kollektor ist.

6. Vorrichtung nach einem der Ansprüche 1 bis 5, **dadurch gekennzeichnet, dass** die Steuerparametergruppe des Motors minimale und maximale Rotationsgeschwindigkeit, genehmigte Rotationsrichtungen, ein maximal zulässiges Moment in einer Rotationsrichtung im Uhrzeigersinn, ein maximal zulässiges Moment in einer Rotationsrichtung entgegen dem Uhrzeigersinn und eine Beibehaltungsaktion des Moments oder des Stopps des Motors, wenn das maximale Moment erreicht ist, umfasst.

7. Vorrichtung nach einem der Ansprüche 1 bis 6, **dadurch gekennzeichnet, dass** die Rotationssteuermittel (8, 10) des Motors im Instrument (1) integriert sind und aus der Gruppe ausgewählt sind, die einen Druckknopf, einen Drehknopf und ein Gate umfasst.

8. Vorrichtung nach einem der Ansprüche 1 bis 6, **dadurch gekennzeichnet, dass** die Rotationssteuermittel des Motors (2, 52) in ein Pedal integriert sind, das mit einem flexiblen elektrischen Anschluss mit dem Instrument verbunden ist.

9. Vorrichtung nach einem der Ansprüche 4 bis 8, **dadurch gekennzeichnet, dass** die gesicherten manuellen Auswahlmittel einen Cursor (17), eine Gruppe von Piktogrammen (18), die jeweils ein Merkmal eines Werkzeugs darstellen, und einen Kranz (19, 56), der etwa gemäß der Richtung der Antriebsachse (4, 54) des Motors auf der gegenüberliegenden Seite der Montage des Handteils rotierend montiert ist, wobei die Mittel ausgebildet sind, damit der rotierende Kranz (19, 56) den Cursor (17) sukzessiv über jedem Piktogramm (18) ausrichtet, um manuell das Merkmal des Werkzeugs zu wählen und die gewählte Steuerparametergruppe einzustellen, wobei die gesicherten Auswahlmittel ferner ausgebildet sind, um mit einer Handhabung durch einen Rechtshänder oder einen Linkshänder kompatibel zu sein.

10. Vorrichtung nach einem der Ansprüche 1 bis 9, **dadurch gekennzeichnet, dass** das Instrument (1) ferner einen Handgriff umfasst, der etwa senkrecht zur Antriebsachse ist.

11. Vorrichtung nach einem der Ansprüche 1 bis 9, **dadurch gekennzeichnet, dass** das Instrument (50) die Form eine Stifts hat.

12. Vorrichtung nach einem der Ansprüche 1 bis 11, **dadurch gekennzeichnet, dass** sie Infrarot-Navigationsmittel umfasst, die ausgebildet sind, um eine 2D-Position eines Implantats zu senden.

13. Vorrichtung nach einem der Ansprüche 1 bis 12, **dadurch gekennzeichnet, dass** die elektrische Versorgungsquelle über einen flexiblen elektrischen Anschluss (12) bereitgestellt wird, der mit dem Instrument verbunden ist.

14. Vorrichtung nach einem der Ansprüche 1 bis 12, **dadurch gekennzeichnet, dass** die elektrische Versorgungsquelle von einer lösbaren Batterie bereitgestellt wird, die auf dem Instrument montiert ist.

## Claims

1. A surgical device including a hand instrument (1, 50) provided with a rotary motor (2, 52), a driving shaft (4, 54) on which is mounted a removable hand part comprising a tool holder (20, 30, 40) and a tool, said instrument being further provided
- with a servo-controlled electronic module of said motor comprising a digital electronic circuit,
- with means for controlling rotation of said motor (2, 52),
- with means for automatically detecting the type of the mounted tool holder,
- and with an electric power supply (12),
said digital electronic circuit being provided with at least one memory containing at least two sets of servo-control parameters of said motor (2, 52), each set of servo-control parameters being associated with a tool holder (20, 30, 40), said digital electronic circuit being laid out for automatically selecting the set of servo-control parameters according to the type of tool holder detected by said automatic detection means,
**characterized in that** the means for automatic detection of the type of tool holder include
- at least one ergot (22, 32) laid out on the tool holder for mechanically cooperating with at least one recess (16) of said motor (2, 52), when said tool holder (20, 30, 40) is mounted on the driving shaft (4, 54),
- at least one magnetized element laid out on said tool holder (20, 30, 40) for occupying a first position associated with a type of tool holder (20, 30, 40), said first position being referenced to the position of said ergot (22, 32),
- at least one sensor (15) laid out on said motor (2, 52) for occupying a second position referenced to the position of said recess,
and **in that** the electronic circuit is laid out for processing output signals of said sensor for detecting the type of tool holder mounted according to the relative position of said magnetized element with said sensor.

2. The device according to claim 1, **characterized in that** each set of servo-control parameters is associated with a tool holder (20, 30, 40) and comprises a minimum and maximum speeds of rotation, authorized directions of rotation, of the maximum torque allowed in a clockwise direction of rotation, of the maximum torque allowed in an anticlockwise direction of rotation and of the action for maintaining the torque or stopping the motor when the maximum torque is attained.

3. The device according to any of claims 1 or 2, **characterized in that** the sensor is a Hall effect or magnetoresistive sensor.

4. The device according to any of claims 1 to 3, **characterized in that** it further comprises secured manual selection means laid out for adjusting the automatically selected set of servo-control parameters.

5. The device according to any of claims 1 to 4, **characterized in that** said motor (2, 52) is an electric motor without any collector.

6. The device according to any of claims 1 to 5, **characterized in that** said set of servo-control parameters of the motor comprises minimum and maximum speeds of rotation, authorized directions of rotation, an authorized maximum torque in a clockwise direction of rotation, an authorized maximum torque in an anti-clockwise direction of rotation, and an action for maintaining the torque or stopping the motor when the maximum torque is attained.

7. The device according to any of claims 1 to 6, **characterized in that** the means (8, 10) for controlling the rotation of said motor are integrated to the instrument (1) and are selected from the group comprising a pushbutton, a rotary button and a trigger.

8. The device according to any of claims 1 to 6, **characterized in that** the means for controlling the rotation of said motor (2, 52) are integrated to a pedal connected through a flexible electric connector to the instrument.

9. The device according to any of claims 4 to 8, **characterized in that** the manual selection secured means comprise a cursor (17), a set of pictograms (18) each representing a characteristic of a tool and a crown (19, 56) mounted in rotation substantially along the direction of the driving axis (4, 54) of the motor on the side opposite to the mounting of the hand part, said means being laid out so that the crown (19, 56) in rotation successively aligns the cursor (17) on each pictogram (18) so as to manually select the characteristic of the tool and adjust the selected set of servo-control parameters, the secured selection means being further laid out so as to be compatible with handling by a right-handed person or left-handed person.

10. The device according to any of claims 1 to 9, **characterized in that** the instrument (1) further comprises a handle substantially perpendicular to the driving axis.

11. The device according to any of claims 1 to 9, **characterized in that** the instrument (50) has the shape of a pencil.

12. The device according to any of claims 1 to 11, **characterized in that** it comprises navigation IR means laid out for emitting a 2D position of an implant.

13. The device according to any of claims 1 to 12, **characterized in that** the electric power supply source is provided via a flexible electric collection (12) connected to the instrument.

14. The device according to any of claims 1 to 12, **characterized in that** the electric power supply source is provided by a removable battery mounted on the instrument.
